# DEMANDE DE BREVET EUROPEEN

(11) **EP 1 506 767 A1**
(43) Date de publication de la demande: **16.02.2005**
(21) Numéro de dépôt: 04291766.6
(22) Date de dépôt: 12.07.2004
(51) Int. Cl.: A61K 7/06

(54) **Utilisation d'une amine carbonylée pour stimuler la pousse des fibres kératiniques et/ou freiner leur chute**

(30) Priorité: 11.08.2003 FR 0350424
(71) Demandeur: L'OREAL, 75008 Paris (FR)
(72) Inventeur: Cavezza, Alexandre, 93290 Tremblay-en-France (FR); Dalko, Maria, 91190 Gif S/Yvette (FR)
(74) Mandataire: Wattremez, Catherine

(57) **Abrégé**

L'invention se rapporte à l'utilisation d'au moins une amine carbonylée de formule (I) suivante ou de l'un de ses sels physiologiquement acceptables, dans une composition de soin des cheveux ou des cils, à application topique, destinée à stimuler leur pousse et/ou freiner leur chute : 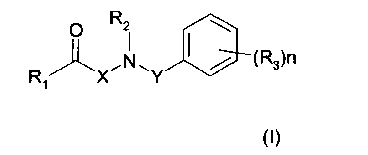 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
   - un radical alkyle, linéaire ou ramifié, saturé ou insaturé, en C₁-C₆, ou un radical aryle,
   - un atome d'halogène, ou
   - un groupe choisi parmi -CN, -OR, -SR, -NRR', -COR, -COOR, -CONRR',
   - NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR,-COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', - NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

## Description

La présente invention se rapporte à l'utilisation d'une amine carbonylée dans une composition de soin ou de maquillage des fibres kératiniques humaines, à application topique, destinée à induire et/ou stimuler la croissance des fibres kératiniques et/ou freiner leur chute. Elle se rapporte également à un procédé de traitement cosmétique, destiné à stimuler la croissance des fibres kératiniques et/ou freiner leur chute.

Plus spécialement, l'invention a trait à l'utilisation d'une quantité efficace d'une amine carbonylée dans une composition de soin ou de maquillage des cheveux ou des cils, destinée à augmenter leur densité et/ou améliorer leur aspect.

La croissance des cheveux et leur renouvellement sont principalement déterminés par l'activité des follicules pileux et de leur environnement matriciel. Leur activité est cyclique et comporte essentiellement trois phases à savoir la phase anagène, la phase catagène et la phase télogène.

A la phase anagène (phase active ou de croissance), qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.

La phase terminale ou phase télogène, qui dure quelques mois, correspond à une phase de repos du follicule et le cheveu finit par tomber. A la fin de cette période de repos, un nouveau follicule est régénéré, sur place, et un autre cycle recommence.

La chevelure se renouvelle donc en permanence et sur les 150 000 cheveux environ que comporte une chevelure, 10% environ sont au repos et seront remplacés en quelques mois.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

A l'âge adulte, le système vasculaire de la peau est parachevé et ne se modifie plus sauf au niveau des follicules pileux où il subit d'importantes variations à chaque cycle pilaire. Les follicules pileux sont en effet une structure cutanée richement innervée et très vascularisée. Le phénomène de développement de la microcirculation des follicules pileux est appelé angiogénèse. Au début de chaque phase anagène, il est nécessaire de développer une forte activation de l'angiogénèse afin de re-développer le microréseau vasculaire périfolliculaire. L'involution de ce microréseau et la disparition des capillaires sanguins de la papille dermique vont de pair avec le changement de phase et le passage en phase catagène. A ce stade, les microvaisseaux sanguins collapsent et disparaissent.

Parallèlement, dans les zones alopéciques, une fibrose périfolliculaire s'installe, les follicules se miniaturisent cycle après cycle et progressivement, la vascularisation spécifique des bulbes s'amoindrie.

Le phénomène d'angiogénèse observé au cours de la phase anagène dépend de nombreux facteurs trophiques, de cytokines ou d'autres molécules biologiquement actives apportées par la circulation sanguine ou localement produites en particulier par les fibroblastes de la papille dermique ou les kératinocytes du bulbe capillaire. Parmi ces facteurs trophiques on peut citer le facteur de croissance des cellules endothéliales (appelé également vascular endothelial growth factor (VEGF) en terminologie anglo-saxonne). Ce facteur est essentiel pour l'angiogénèse et augmente la perméabilité vasculaire. Des études ont montré que l'expression de ce facteur était augmentée au cours de la phase anagène du cycle pilaire. Ainsi, ce facteur contribue au maintien d'une microvascularisation fonctionnelle autour du follicule pileux, et notamment de la base du bulbe et de la papille dermique, ainsi qu'à l'apport de nutriments nécessaires à la bonne croissance du cheveu.

La microcirculation périfolliculaire joue donc un rôle primordial dans le processus de croissance pilaire en apportant les facteurs et les nutriments nécessaires à la croissance de ce follicule.

La chute des cheveux peut être fortement accentuée et les cycles de renouvellement des follicules peuvent être fortement perturbés dans certaines dermatoses du cuir chevelu à caractéristique inflammatoire, telles que par exemple le psoriasis ou les dermatites séborrhéiques.

D'autres causes peuvent entraîner une perte importante, temporaire ou définitive, des cheveux. Il peut s'agir de chute et d'altération des cheveux au décours d'une grossesse (post partum), au cours d'états de dénutrition ou malnutrition, de stress physiologique, de déséquilibres alimentaires ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers.

Il peut s'agir également d'une alopécie, qui est essentiellement due à une perturbation du renouvellement capillaire entraînant dans un premier temps l'accélération de la fréquence des cycles au détriment de la qualité des cheveux, puis de leur quantité. Il se produit alors un appauvrissement progressif de la chevelure et une miniaturisation progressive des cheveux, conjointement à un isolement des bulbes par une progression de l'épaisseur de la matrice collagénique périfolliculaire et de la gaine conjonctive externe. La revascularisation est donc rendue plus difficile cycle après cycle. Les cycles de croissance successifs aboutissent à des cheveux de plus en plus fins et de plus en plus courts, se transformant peu à peu en un duvet non pigmenté. Des zones sont touchées préférentiellement, notamment les golfes temporaux ou frontaux chez l'homme et, chez la femme, on constate une alopécie diffuse du vertex.

De par le rôle primordial de la microcirculation périfolliculaire énoncé plus haut, tout défaut de cette dernière entraînera une diminution de l'apport des éléments nutritifs et gazeux (Oxygène notamment) nécessaires à la croissance pilaire conduisant à des troubles de la croissance du cheveu et la mise en place progressive d'une alopécie.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Dans certaines dermatoses du cuir chevelu à caractéristique inflammatoire, telles que par exemple le psoriasis ou les dermatites séborrhéiques, la chute des cheveux peut être fortement accentuée ou entraîner des cycles des follicules fortement perturbés.

De façon générale, tout facteur entraînant une augmentation de l'apport sanguin au niveau du follicule pileux soit en activant l'angiogénèse, soit en s'opposant à sa régression, soit encore en agissant sur les microvaisseaux pour limiter leur constriction, aura un effet bénéfique sur l'apport énergétique nécessaire à la bonne croissance de ce même follicule.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute. L'une des voies explorées est justement le maintien de la vascularisation autour du follicule pileux.

Ainsi, un des composés connu pour maintenir la vascularisation périfolliculaire est le vérapamil, qui est un antagoniste puissant des canaux calciques de type L. Le vérapamil et d'autres antagonistes de canaux calciques comme le diltiazem et la nifédipine sont décrits comme étant actifs dans le traitement de la chute des cheveux, en particulier par leurs effets sur la microcirculation (confère les documents de Shiseido JP88/062680 et de Coppe J. BE/89/000305).

En outre, il existe des documents décrivant l'utilisation de donneurs de NO (monoxyde d'azote) pour application sur le cuir chevelu, pour stimuler la croissance de cheveux en agissant sur la microcirculation du cuir chevelu. Ainsi, le document brevet de Proctor (EP 0327263) décrit l'utilisation de composés producteurs du radical NO, en association avec des agents réducteurs, des anti-oxydants et avec des piégeurs de radical hydroxyle. Un autre document brevet de E. Fossel (WO 99/13717) décrit l'utilisation de l'arginine et de ses dérivés, comme substrat de NO-synthase, pour la formation *in vivo* de NO et leur utilisation (entre autre) dans le traitement de l'alopécie. Un autre document brevet de Shiseido (JP-A-07316023) décrit également l'utilisation de l'arginine et de ses dérivés dans le traitement de l'alopécie.

Ces substances connues présentent des effets indésirables. En particulier, elles présentent des activités multiples, pouvant perturber l'équilibre ionique et physiologique des cellules cutanées. Autrement dit, leur activité multiple rend difficile leur contrôle d'action sur les cellules.

Le présente invention se rapporte à l'utilisation d'amines carbonylées particulières permettant de remédier aux inconvénients ci-dessus. Ces amines présentent, entre autre, une activité locale, spécifique. Elles assurent une revascularisation du follicule pileux, de sa région périphérique et/ou du cheveu après chaque cycle de croissance. Ces amines sont d'une façon surprenante dotées d'une activité favorable à l'amélioration de la densité des fibres kératiniques humaines. Ainsi, ces composés ont un effet bénéfique sur la pousse des cheveux humains mais aussi sur la pousse des cils et de certains poils humains.

La présente invention a donc pour objet l'utilisation notamment cosmétique, comme agent pour induire et/ou stimuler la croissance des fibres kératiniques humaines notamment les cils et les cheveux et/ou freiner leur chute et/ou augmenter leur densité, d'une quantité efficace d'au moins une amine carbonylée répondant à la formule générale (I) suivante ou à son sel d'addition avec un acide : 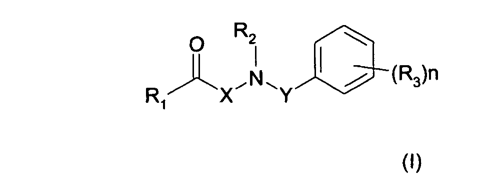 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
   - un radical alkyle, linéaire ou ramifié, saturé ou insaturé, en C₁-C₆, ou un radical aryle,
   - un atome d'halogène, ou
   - un groupe choisi parmi -CN, -OR, -SR, -NRR', -COR, -COOR, -CONRR', - NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR,-COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', -NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

Par augmenter la densité des fibres kératiniques, et notamment la densité capillaire, on entend augmenter le nombre de fibres kératiniques, notamment de cheveux, par cm² de peau d'où émergent lesdites fibres telle que le cuir chevelu.

L'invention se rapporte encore à l'utilisation cosmétique d'au moins une amine carbonylée de formule (I) ou de l'un de ses sels d'addition avec un acide, dans une composition cosmétique de soin et/ou de maquillage des fibres kératiniques d'être humain, pour réduire la chute des fibres kératiniques et/ou augmenter leur densité.

Elle a encore pour objet l'utilisation d'au moins une amine carbonylée de formule (I) ou de l'un de ses sels d'addition avec un acide, pour la préparation d'une composition de soin et/ou de traitement des fibres kératiniques d'être humain, destinée à induire et/ou stimuler la croissance des fibres kératiniques et/ou freiner leur chute et/ou augmenter leur densité.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

Aussi, l'invention se rapporte encore à l'utilisation cosmétique d'au moins une amine carbonylée de formule (I) ou de l'un de ses sels d'addition avec un acide, dans une composition cosmétique de soin capillaire d'être humain pour traiter (réduire) la chute des cheveux et/ou augmenter leur densité et/ou traiter l'alopécie andro-chrono-génétique. Elle a encore pour objet l'utilisation d'au moins une amine carbonylée de formule (I) ou d'un de ses sels d'addition avec un acide pour la préparation d'une composition capillaire pour être humain, destinée à induire et/ou stimuler la croissance des cheveux et/ou freiner leur chute et/ou augmenter leur densité et/ou traiter l'alopécie androgénique. En particulier, cette composition permet de maintenir en bon état la chevelure et/ou de lutter contre la chute naturelle des cheveux plus spécialement des hommes.

L'invention se rapporte encore à l'utilisation cosmétique d'au moins une amine carbonylée de formule (I) ou d'un de ses sels d'addition avec un acide, dans une composition cosmétique de soin capillaire d'être humain pour traiter l'alopécie d'origine naturelle et en particulier androgénique ainsi qu'à l'utilisation d'au moins une amine carbonylée de formule (I) ou d'un de ses sels d'addition avec un acide pour la préparation d'une composition de soin capillaire d'être humain, destinée à traiter l'alopécie d'origine naturelle et en particulier androgénique.

L'invention a encore pour objet l'utilisation cosmétique d'au moins une amine carbonylée de formule (I) ou d'un de ses sels d'addition avec un acide, dans une composition cosmétique de soin et/ou de maquillage des cils d'être humain, pour induire et/ou stimuler la croissance des cils et/ou augmenter leur densité ainsi que l'utilisation d'au moins une amine carbonylée de formule (I) ou d'un de ses sels d'addition avec un acide, pour la préparation d'une composition de soin et/ou de traitement des cils d'être humain, destinée à induire et/ou stimuler la croissance des cils et/ou augmenter leur densité. Cette composition permet ainsi de maintenir en bon état les cils et/ou améliorer leur état et/ou leur aspect.

L'invention a encore pour objet l'utilisation d'au moins une amine carbonylée de formule (I) ou de l'un de ses sels d'addition avec un acide, pour la fabrication d'une composition destinée à traiter les désordres liés à une réduction de la microcirculation ou vascularisation cutanée et notamment du follicule pileux chez l'être humain.

Dans la formule (I), les radicaux alkyle peuvent notamment être choisis, selon le cas, parmi les radicaux : méthyle, éthyle, n-propyle, isopropyle, n-butyle, isobutyle, tert-butyle, pentyle, hexyle, heptyle, octyle, nonyle, décyle, undécyle et dodécyle.

Par ailleurs, le radical aryle peut être choisi parmi le radical benzyle et le radical phényle.

La chaîne alkylène ou alkénylène est en particulier une chaîne méthylène, éthylène, triméthylène, tétraméthylène, pentaméthylène, vinylène ou propénylène.

L'atome d'halogène peut être un atome de fluor, de chlore ou de brome.

Selon un mode de réalisation de l'invention, l'amine de formule (I) particulière ou un de ses sels d'addition avec un acide est tel que l'une au moins des conditions suivantes est satisfaite :
- R₁ est un radical alkyle, linéaire ou ramifié, saturé ou insaturé, en C₁-C₅,
- R₂ est un atome d'hydrogène ou un radical alkyle saturé, linéaire ou ramifié, en C₁-C₈, éventuellement substitué par un groupe oxo,
- R₃ est un radical alkyle, linéaire ou ramifié, saturé ou insaturé, en C₁-C₆ ou un groupe -OR, où R est un atome d'hydrogène ou un radical alkyle, linéaire ou ramifié, saturé, en C₁-C₄,
- n est égal à 0, 1 ou 2,
- X est une chaîne alkylène en C₁-C₄, par exemple une chaîne éthylène,
- Y est une chaîne alkylène en C₁-C₅, par exemple en C₁-C₃.

En particulier, toutes les conditions ci-dessus sont satisfaites.

Des exemples d'amines de formule (I) auxquelles s'applique l'invention comprennent les composés ayant l'une des formules suivantes :

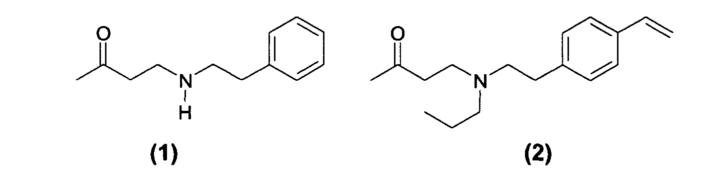

D'autres exemples d'amines de formule (I) auxquelles s'applique l'invention comprennent les composés répondant à la formule générale (II) ci-dessous : 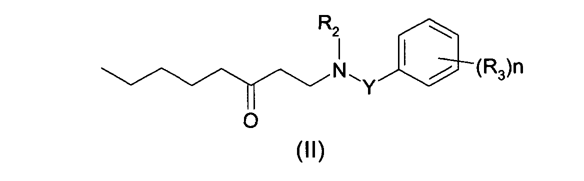 dans laquelle R₂, R₃, n et Y ont la signification indiquée précédemment pour la formule (I) générale ou particulière.

Selon un mode de réalisation de l'invention, l'amine carbonylée de formule (II) ou son sel d'addition avec un acide satisfait à l'une au moins des conditions suivantes :
- R₂ désigne un atome d'hydrogène ou un radical alkyle, linéaire ou ramifié, saturé ou insaturé, en C₁-C₈, éventuellement substitué par un groupe oxo ;
- R₃ désigne
   - un radical alkyle linéaire, ramifié ou cyclique, saturé ou insaturé, en C₁-C₆, ou un groupe aryle,
   - un atome d'halogène, ou
   - un groupe -CN, -OR, -SR, -NRR', -COR, -COOR, -CONRR' ou -CF₃ ;
- Y est une chaîne alkylène ou alkénylène en C₁-C₂₀, éventuellement substituée par au moins l'un des groupements -OR, -SR, -NRR', -COOR, -CO-NRR', -NR-CO-R', F et CF₃ et/ou par un groupe aryle,
- n est en entier allant de 0 à 5 ;
où R et R' désignent indépendamment un atome d'hydrogène ou un radical alkyle en C₁-C₄, linéaire ou ramifié.

Selon un mode de réalisation particulier de l'invention, l'amine de formule (II) ci-dessus, satisfait à l'une au moins des conditions suivantes :
- R₂ est un atome d'hydrogène ou un radical alkyle, linéaire ou ramifié, saturé, en C₁-C₈, éventuellement substitué par un groupe oxo, de préférence un radical alkyle en C₁-C₃,
- R₃ est un groupe -OR, où R est un atome d'hydrogène ou un radical alkyle, linéaire ou ramifié, saturé, en C₁-C₄,
- n est égal à 0, 1 ou 2, de préférence 0,
- Y est une chaîne alkylène en C₁-C₅, de préférence en C₁-C₃.

Des exemples d'amines de formule (II) sont notamment des composés répondant aux formules **(3)** à **(7)** ci-dessous :

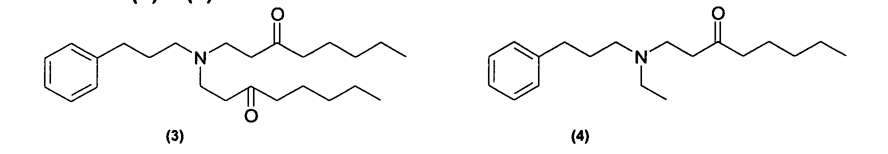

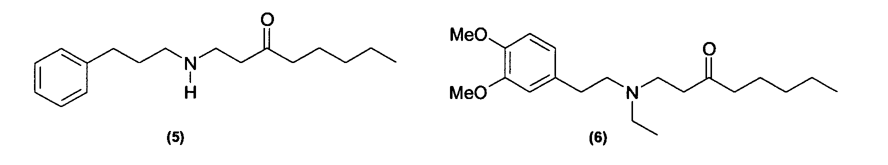

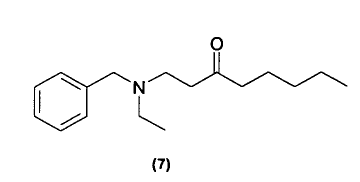

Les amines de formule (I) auxquelles s'applique l'invention peuvent être utilisées seules ou en mélange en toutes proportions. En outre, elles peuvent se présenter sous forme de sels physiologiquement acceptables obtenus par addition de l'amine de formule (I) [voire (II)] avec un acide organique ou inorganique.

L'acide inorganique est choisi notamment parmi les acides chlorhydrique, sulfurique, nitrique et phosphorique et l'acide organique est choisi en particulier parmi les acides malonique, succinique, fumarique, lactique, glycolique, acétique, citrique et tartrique.

Les amines de formule (I) [voire (II)] peuvent notamment être préparées suivant un procédé analogue à celui décrit à l'Exemple 1.

A la connaissance du demandeur, aucun document de l'art antérieur ne décrit ni ne suggère que les amines de formule (I) ou leurs sels d'addition avec un acide ont la propriété d'induire et/ou de stimuler la croissance des fibres kératiniques et/ou de freiner leur chute ni que ces amines ou leurs sels peuvent être utilisés par voie topique pour augmenter la densité de ces fibres.

La quantité efficace d'une amine carbonylée de formule (I) ou de l'un de ses sels d'addition avec un acide, correspond à la quantité nécessaire pour obtenir le résultat désiré (en particulier à savoir augmenter la densité des fibres kératiniques ou favoriser leur pousse). L'homme du métier est donc en mesure d'évaluer cette quantité efficace qui dépend de la nature de l'amine utilisée, de la personne à laquelle on l'applique, et du temps de cette application.

Dans la suite du texte, et sauf indication contraire, les quantités des différents ingrédients de la composition sont données en pourcentage en poids par rapport au poids total de la composition.

Pour donner un ordre de grandeur, selon l'invention, l'amine de formule (I) [voire (II)] ou de l'un de ses sels d'addition avec un acide, peut être utilisé en une quantité représentant de 10⁻³ % à 10% du poids total de la composition et préférentiellement en une quantité représentant de 10⁻² % à 5% du poids total de la composition, par exemple de 0,5 % à 2 %.

La composition de l'invention peut être à usage cosmétique ou pharmaceutique (notamment dermopharmaceutique). Préférentiellement, la composition de l'invention est à usage cosmétique. Aussi, la composition doit contenir un milieu physiologiquement acceptable non toxique et susceptible d'être appliqué sur la peau, y compris le cuir chevelu et les paupières, et sur les fibres kératiniques comme les cheveux et les cils. Par "cosmétique", on entend au sens de l'invention une composition d'aspect, d'odeur et de toucher agréables.

L'amine de formule (I), salifiée ou non, peut être utilisée dans une composition à ingérer, injecter ou appliquer sur la peau ou les fibres kératiniques (sur toute zone cutanée ou fibres à traiter).

Les amines et leurs sels auxquels s'appliquent peuvent être utilisés par la voie orale en une quantité de 0,1 à 300 mg par jour, 5 à 10mg/j.

Une composition préférée de l'invention est une composition à usage cosmétique et en particulier d'application topique sur la peau et les fibres kératiniques, et plus spécialement sur le cuir chevelu, les cheveux et les cils.

Cette composition peut se présenter sous toutes formes galéniques connues adaptées au mode d'utilisation.

Pour une application topique sur la peau et les fibres kératiniques, y compris le cuir chevelu, la composition peut avoir la forme d'une solution ou suspension aqueuse, alcoolique, hydro-alcoolique ou huileuse, d'une émulsion ou dispersion de consistance plus ou moins fluide et notamment liquide ou semi-liquide, obtenue par dispersion d'une phase grasse dans une phase aqueuse (H/E) ou inversement (E/H), d'une dispersion ou émulsion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique.

On peut également envisager une composition sous forme de mousse ou encore sous forme de spray ou d'aérosol comprenant alors un agent propulseur sous pression.

La composition peut ainsi se présenter sous forme d'une lotion, sérum, lait, crème H/E ou E/H, gel, onguent, pommade, poudre, baume, patch, tampon imbibé, savon, pain, mousse.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Pour une composition à usage par injection, la composition peut se présenter sous forme de lotion aqueuse ou de suspension huileuse par exemple sous forme de sérum. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire, de mascara capillaire ou pour cils.

Les quantités des différents constituants du milieu physiologique de la composition selon l'invention sont celles généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (I) ou (II) ainsi que de celle des éventuels ingrédients additionnels, pour obtenir 100% en poids. En pratique la phase aqueuse représente de 5 % à 99,9% en poids.

La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique. En plus des ces huiles, la phase grasse peut, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Les émulsionnants et coémulsionnants utilisés pour l'obtention d'une composition sous forme d'émulsion sont ceux généralement utilisés dans les domaines cosmétique et pharmaceutique. Leur nature est, en outre, fonction du sens de l'émulsion. En pratique, l'émulsionnant et éventuellement le coémulsionnant sont présents, dans la composition, en une proportion allant de 0,1 % à 30 % en poids, de préférence de 0,5 à 20 % en poids et mieux de 1 à 8%. L'émulsion peut, en outre, contenir des vésicules lipidiques et notamment des liposomes.

Lorsque la composition est sous forme d'une solution ou d'un gel huileux, la phase grasse peut représenter plus de 90 % du poids total de la composition.

Avantageusement, pour une application capillaire, la composition est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara, la composition est en particulier une dispersion de cire-dans-eau ou de cire-dans-huile, une huile gélifiée, un gel aqueux. Elle peut être pigmentée ou non.

La composition de l'invention peut comprendre, en outre, d'autres ingrédients usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les agents bloqueurs d'U.V. comme les filtres solaires, les polymères filmogènes, les actifs cosmétiques et pharmaceutiques à action bénéfique pour la peau ou les fibres kératiniques, autres que les composés de formule (I) ou (II) (comme les vitamines), leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20% et par exemple de 0,1 à 10 %. Ces adjuvants, selon leur nature, peuvent être introduits dans la phase grasse, dans la phase aqueuse et/ou dans les vésicules lipidiques et notamment des liposomes.

Bien entendu l'homme du métier veillera à choisir les éventuels additifs complémentaires et/ou leur quantité de telle manière que les propriétés avantageuses de la composition selon l'invention, notamment à savoir l'augmentation de la densité des fibres kératiniques, ne soient pas ou substantiellement pas, altérées par l'adjonction envisagée.

Comme solvants utilisables dans l'invention, on peut citer les alcools inférieurs en C₂ à C₈ comme l'éthanol, l'isopropanol, le propylène glycol et certaines huiles cosmétiques légères comme les alcanes en C₆ à C₁₆.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, de soja, alcool ou acide gras), les huiles d'origine animale (perhydrosqualène), les huiles de synthèse (esters d'acide gras, huile de Purcellin), les huiles siliconées (polydiméthylsiloxanes linéaires ou cycliques, phényltriméthicones) et les huiles fluorées (perfluoropolyéthers). Comme cires, on peut citer les cires siliconées, les cires d'abeille, de candellila, de riz, de carnauba, de paraffine ou de polyéthylène.

Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E. L'émulsionnant et le coémulsionnant sont présents, dans la composition, en une proportion allant de 0,3% à 30% en poids, et de préférence de 0,5 à 20% en poids par rapport au poids total de la composition.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.

Comme actif cosmétique ou pharmaceutique autre que les amines de formule (I), la composition peut contenir un actif additionnel hydrophile choisi parmi les protéines ou les hydrolysats de protéine, les acides aminés, les polyols, l'urée, l'allantoïne, les sucres et les dérivés de sucre, les vitamines hydrosolubles, les extraits végétaux (ceux d'Iridacées ou de soja) et les hydroxy-acides (acide de fruit, acide salicylique) ; et/ou un actif additionnel lipophile choisi parmi le rétinol (vitamine A) et ses dérivés notamment ester (palmitate de rétinol), le tocophérol (vitamine E) et ses dérivés notamment ester (acétate ou palmitate de tocophérol), les acides gras essentiels, les céramides, les huiles essentielles, les dérivés de l'acide salicylique comme le n-octanoyl-5 salicylique, les esters des hydroxy acides, les phospholipides comme la lécithine, leurs mélanges.

Selon un mode particulier de réalisation de l'invention, on peut associer à l'amine carbonylée de formule (I) ou à l'un de ses sels, au moins un composé additionnel favorisant la repousse et/ou limitant la chute des fibres kératiniques (cheveux ou cils). Ces composés additionnels sont notamment choisis parmi les inhibiteurs de lipoxygénase tels que décrits dans EP 0648488, les inhibiteurs de bradykinine décrits notamment dans EP 0845700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1175891 et EP 1175890, WO 01/74307, WO 01/74313, WO 01/74314, WO 01/74315 ou WO 01/72268, leurs mélanges.

Comme autres composés additionnels favorisant la pousse du cheveu pouvant être présents dans la composition selon l'invention on peut citer les vasodilatateurs, les antiandrogènes, les cyclosporines et leurs analogues, les antimicrobiens et antifongiques, les anti-inflammatoires, les rétinoïdes, seuls ou en mélange.

Les vasodilatateurs utilisables sont notamment les agonistes des canaux potassium incluant le minoxidil ainsi que les composés décrits dans les brevets US 3 382247, 5 756092, 5 772990, 5 760043, 5 466694, 5 438058, 4 973474, la cromakalim, le nicorandil et le diaxozide, seuls ou en association.

Les antiandrogènes utilisables incluent notamment les inhibiteurs stéroïdiens ou non stéroïdiens de 5α-réductase, comme le finastéride et les composés décrits dans US 5 516779, l'acétate de cyprostérone, l'acide azélaïque, ses sels et ses dérivés et les composés décrits dans US 5 480913, le flutamide, l'oxendolone, la spironolactone, le diéthylstilbestrol et les composés décrits dans les brevets US 5 411981, 5 565467 et 4 910226.

Les composés antimicrobiens ou antifongiques peuvent être choisis parmi les dérivés du sélénium, l'octopirox, le triclocarban, le triclosan, le pyrithione zinc, l'itraconazole, l'acide asiatique, l'hinokitiol, la mipirocine, les tétracyclines, notamment l'érythromycine et les composés décrits dans EP 0680745, le chlorhydrate de clinycine, le peroxyde de benzoyle ou de benzyle, la minocycline et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en C₁-C₆ comme les nicotinates de méthyle ou d'hexyle.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'α-bisabolol, la benzydamine, l'acide salicylique et les composés décrits dans EP 0770399, WO 94/06434 et FR 2268523.

Les rétinoïdes peuvent être choisis parmi l'isotrétinoïne, l'acitrétine et le tazarotène.

Comme autres composés additionnels actifs pour favoriser la pousse et/ou limiter la chute des cheveux utilisables en association avec le composé de formule (I), salifié ou non, on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les benzophénones et l'hydantoïne, l'acide rétinoïque ; les vitamines comme la vitamine D, les analogues de la vitamine B12 et le panthoténol ; les triterpènes comme l'acide ursolique et les composés décrits dans US 5529769, US 5468888, US 5631282 ; les agents antiprurigineux comme la thénaldine, la triméprazine ou la cyproheptadine ; les antiparasitaires, en particulier le métronidazole, le crotamiton ou les pyréthrinoïdes ; les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les antagonistes du récepteur FP (récepteur aux prostaglandines du type F) tels que le latanoprost, le bimatoprost, le travoprost, l'unoprostone ; les inhibiteurs de 15-hydroxyprostaglandine désydrogénase ; leurs mélanges.

On peut également envisager que la composition comprenant au moins une amine de formule (I), salifiée ou non soit sous forme liposomée, telle que notamment décrite dans le document WO 94/22468. Ainsi, le composé encapsulé dans les liposomes peut être délivré sélectivement au niveau du follicule pileux.

La composition à laquelle s'applique l'invention peut être appliquée sur les zones alopéciques du cuir chevelu et des cheveux d'un individu, et éventuellement laissée en contact plusieurs heures et éventuellement rincée.

On peut, par exemple, appliquer la composition contenant une quantité efficace d'une amine carbonylée de formule (I), salifiée ou non, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des fibres kératiniques humaines et/ou de la peau d'où émergent ces fibres dont le cuir chevelu et les paupières, destiné à stimuler la croissance des fibres kératiniques humaines telles que les cheveux et les cils d'être humain et/ou freiner leur chute, caractérisé par le fait qu'il consiste à appliquer sur les fibres kératiniques humaines et/ou la peau d'où émergent les fibres, une composition cosmétique comprenant une quantité efficace d'au moins une amine carbonylée de formule (I) ou d'un de ses sels d'addition avec un acide, à laisser celle-ci en contact avec les fibres kératiniques et/ou la peau, et éventuellement à rincer les fibres kératiniques et/ou la peau.

Ce procédé de traitement présente les caractéristiques d'un procédé cosmétique dans la mesure où il permet d'améliorer l'esthétique des fibres kératiniques et en particulier des cheveux et des cils en leur donnant une plus grande vigueur et un aspect amélioré. En outre, il peut être utilisé quotidiennement pendant plusieurs mois, sans prescription médicale.

Plus spécialement, la présente invention a pour objet un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu, une composition cosmétique comprenant au moins une amine de formule (I) ou l'un de ses sels d'addition avec un acide, à laisser celle-ci au contact des cheveux et/ou du cuir chevelu, et éventuellement à rincer les cheveux et/ou le cuir chevelu.

L'invention a encore pour objet un procédé de soin cosmétique et/ou de maquillage ces cils humains, en vue d'améliorer leur état et/ou leur aspect, caractérisé en ce qu'il consiste à appliquer une composition de mascara comprenant au moins une amine carbonylée de formule (I) ou l'un de ses sels d'addition avec un acide et à laisser celle-ci au contact des cils. Cette composition de mascara peut être appliquée seule ou en sous-couche d'un mascara pigmenté classique et être éliminée comme un mascara pigmenté classique.

Avantageusement, dans le procédé selon l'invention, on applique sur les zones à traiter du cuir chevelu entre 5 et 500 µl d'une solution ou composition telle que définie précédemment, comprenant de 0,001% à 5 % d'amine de formule (I).

D'autres caractéristiques et avantages de l'invention ressortiront mieux des exemples qui suivent, donnés à titre illustratif et non limitatif. Dans ce qui suit, les proportions sont données en pourcentage massique, sauf indication contraire.

### EXEMPLE 1 : Préparation de la 1-(N-éthyl, (3-phényl-) propylamino) octan-3-one

La 1-(N-éthyl, (3-phényl-) propylamino) octan-3-one peut être préparée suivant un procédé en deux étapes, selon le schéma suivant :

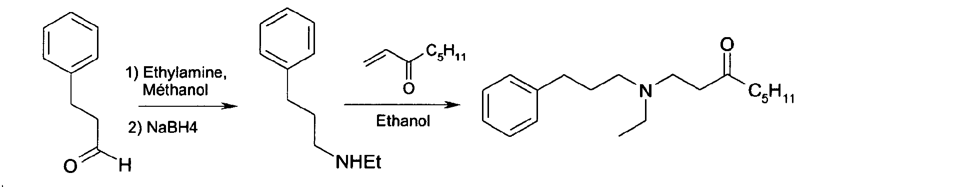

### Etape 1 : synthèse de la N-éthyl, (3-phényl)propylamine

On solubilise 2 g de 3-phényl propionaldéhyde dans 5 ml de méthanol. On ajoute 7,45 g d'éthylamine en solution dans l'éthanol (2 M), puis on laisse réagir pendant 20 heures à température ambiante. On ajoute ensuite lentement 0,68 g de borohydrure de sodium (1,2 eq.) et on laisse agiter pendant 20 heures à température ambiante. Le produit est traité et purifié sur colonne de silice.

On obtient 530 mg de N-éthyl, (3-phényl)propylamine sous forme de produit mono-tache en chromatographie sur couche mince (rendement : 20%). Les résultats de résonance magnétique nucléaire (RMN) et de spectrométrie de masse sont conformes à la structure attendue.

### Etape 2 : synthèse de la 1-(N-éthyl, (3-phényl-) propylamino) octan-3-one

On solubilise 0,4 g de 1-octène-3-one dans 5 ml d'éthanol absolu. On ajoute ensuite 0,49 g du produit résultant de l'étape 1 en solution dans 5 ml d'éthanol absolu. On laisse réagir pendant 15 heures à température ambiante. Le produit est traité et purifié sur colonne de silice. On obtient 300 mg de produit (rendement : 30%). Les résultats de RMN et de spectrométrie de masse sont conformes à la structure attendue.

### EXEMPLE 2 : Mise en évidence de l'effet des composés selon l'invention

### 1°) Propriétés inhibitrices des contractions musculaires

La molécule de l'exemple 1 a été testée à différentes concentrations, à savoir 10, 50 et 100 µM, sur un modèle de coculture nerfs/muscles, qui permet de recréer un arc moteur en innervant des cellules musculaires striées humaines avec des explants de moelle épinière et de ganglions rachidiens d'embryons de rat.

Ce test est prédictif d'un effet inhibiteur des contractions des fibres musculaires.

### a) Protocole

Des cellules musculaires humaines, issues de prélèvements de muscles striés de donneurs sains, sont ensemencées dans des puits de 1,8 cm² de section (boîtes de culture de 24 puits). Après 10 jours de culture, ces cellules forment une monocouche et fusionnent. A ce stade, des explants de moelle épinière d'embryons de rat de 13 jours contenant les ganglions rachidiens sont déposés sur la culture.

La croissance des neurites est visible en dehors de l'explant de moelle épinière après un jour de culture. Les premières contractions des fibres musculaires sont observées après 5 à 6 jours de coculture et après 3 semaines, toutes les fibres musculaires au voisinage des explants se contractent.

Les cocultures sont utilisées après 21 jours, quand les fibres musculaires sont striées et possèdent des jonctions neuromusculaires différenciées matures.

Une fibre musculaire ayant des contractions régulières (au moins 60 contractions par minute) est alors sélectionnée dans trois puits de culture différents et le nombre de contractions est comptabilisé sur 30 secondes. Le composé testé, dilué à 1/1000 dans le DMSO, est ensuite incubé pendant 60 secondes dans ces puits, à la concentration respectivement de 10, 50 et 100 µM. A la fin de l'incubation, le nombre de contractions est à nouveau comptabilisé sur 30 secondes. Le test est réalisé en triplicata.

La toxine botulique est utilisée comme référence et testée à 0,1 et 1 µg/ml.

### b) Résultats

Le composé (4) selon l'invention bloque les contractions des trois fibres musculaires référencées à la concentration respectivement de 10, 50, 100 µM (0,3 µg/ml).

Par comparaison, la toxine botulique diminue fortement la fréquence des contractions après une minute et bloque complètement les contractions après deux heures d'incubation, à la concentration de 1 µg/ml ; en outre, elle a un effet bloquant beaucoup plus faible à une minute mais également complet après deux heures, à une concentration de 0,1 µg/ml.

Ainsi, les amines carbonylées selon l'invention sont des agents dermo-décontractants et cet effet augmente avec leur concentration.

Or il est connu dans la littérature que la jonction neuromusculaire et la microcirculation sont étroitement liées (J. M. Pierzga, Microvascular Research 1994, *48*, 50-67). Il a même été démontré que des molécules inhibitrices de la contraction musculaire influencent la microcirculation (S. R. Inman et *al*. Anesthesia & Analgesia, 1994, 78, 682-6).

Par ailleurs, cette microcirculation est elle-même reconnue pour avoir une influence sur la chute des cheveux (V. A. Medical Hypotheses, 2002, 58, 261-263). En effet des études ont montré que la stimulation du flux sanguin par le minoxidil serait à l'origine de son activité anti-chute (R. C. Wester Journal of Investigative Dermatology. 1984, *82*, 515-517).

### 2°) Propriétés inhibitrices des canaux calcium

Par ailleurs, le demandeur a trouvé que les amines de formule (I) et en particulier de formule (II) sont des inhibiteurs de canaux calcium (ou de flux calcique) comme le vérapamil ou l'alvérine. Or, les inhibiteurs de canaux sont connus comme actifs dans le traitement de la chute des cheveux et sur leur effet bénéfique sur la microcirculation (confère description ci-dessus).

Pour qu'une substance soit reconnue comme un inhibiteur des canaux calcium, elle doit pouvoir diminuer la concentration intracellulaire en calcium ou diminuer la liaison du calcium aux protéines intracellulaires comme par exemple la calmoduline, tel que cela est notamment décrit par Galizzi, J. P. *et al*., J. Biol. Chem. 1987, 262 p 6947 ; par Y. Okamiya et *al*., Eur. J. Pharmacol. 1991, 205, p 49 ; par J. A. Wagner et *al*., J. Neurosci. 1988, 8, p 3354 ; par H. R. Lee et *al*., Life Sci. 1984, 35, p 721; par Schoemaker H. et Lauger S. Eur. J. Pharmacol. 1985, 111, p 273 ou encore I. J. Reynolds et *al*., J. Pharmacol. Exp. Ther. 1986, 237, p 731.

Selon le protocole indiqué dans ces documents, le demandeur a déterminé l'IC_{50Ca2+} d'inhibition de flux calcique des composés (3), (4), (6) et (7), en comparaison avec le vérapamil ; les résultats sont donnés dans le tableau ci-après. IC_{50Ca2+} représente la concentration inhibitrice de 50 % de libération de Ca²⁺.

| ***Composés testés*** | ***IC***_{***50Ca2+***} ***en µM*** | |
|---|---|---|
| | Sur cellules issues de fibroblastes de la peau HDFa | Sur cellules nerveuses SK-N-SH |
| nifédipine | ND* | 29 |
| alvérine | ND* | 30 |
| diltiazem | ND | >45 |
| 3 | 18 | 7 |
| 4 | 30 | 18 |
| 6 | 22 | 32 |
| 7 | 18 | 17 |

De ce tableau, il ressort que les amines auxquelles s'applique l'invention sont bien des inhibiteurs de canaux calcium.

De ces tests et connaissances, on en déduit que les amines carbonylées de formule (I) [voire (II)] présentent une forte probabilité d'avoir un effet bénéfique sur la pousse des cheveux humains et/ou sur le freinage de leur chute et/ou sur l'augmentation de leur densité.

### EXEMPLE 3 : Lotion capillaire

- Composé de l'exemple 1 1,00 g
- Propylène glycol 30,00 g
- Alcool éthylique 40,00 g
- Eau qsp 100,00 g

On applique cette lotion sur le cuir chevelu, une à deux fois par jour, pendant quelques mois, à raison d'1 ml par application, en massant légèrement le cuir chevelu pour faire pénétrer l'actif. La chevelure est ensuite séchée à l'air libre. Cette lotion a une probabilité élevée de diminuer la chute des cheveux et/ou de favoriser leur repousse et/ou d'améliorer leur aspect.

### EXEMPLE 4 : Mascara cire/eau

- Cire d'abeilles 6,00 %
- Cire de paraffine 13,00 %
- Huile de jojoba hydrogénée 2 %
- Polymère filmogène hydrosoluble 3 %
- Stéarate de triéthanolamine 8 %
- Composé de l'exemple 1 1 %
- Pigment noir 5 %
- Conservateur qs
- Eau qsp 100 %

Ce mascara s'applique sur les cils comme un mascara classique avec une brosse à mascara. Il a une probabilité élevée d'améliorer l'aspect des cils.

## Revendications

1. Utilisation d'au moins une amine carbonylée répondant à la formule (I) suivante ou à l'un de ses sels d'addition avec un acide, comme agent pour induire et/ou stimuler la croissance des fibres kératiniques des êtres humains et/ou freiner leur chute et/ou augmenter leur densité, 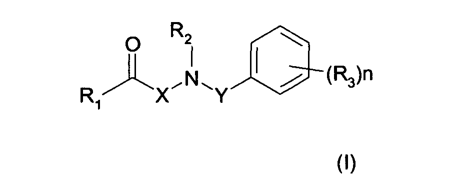 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
- un radical alkyle, linéaire ou ramifié, saturé ou insaturé, en C₁-C₆, ou un radical aryle,
- un atome d'halogène, ou
- un groupe choisi parmi -CN, -OR, -SR, -NRR', -COR, -COOR, -CONRR', - NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR,-COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', -NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

2. Utilisation cosmétique d'au moins une amine carbonylée de formule (I) ou de l'un de ses sels d'addition avec un acide, dans une composition cosmétique de soin et/ou de maquillage des fibres kératiniques d'être humain pour réduire la chute des fibres kératiniques et/ou augmenter leur densité, 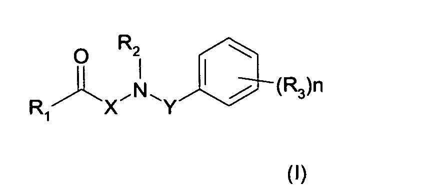 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
- un radical alkyle, linéaire ou ramifié, saturé ou insaturé, en C₁-C₆, ou un radical aryle,
- un atome d'halogène, ou
- un groupe choisi parmi -CN, -OR, -SR, -NRR', -COR, -COOR, -CONRR', -NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR,-COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', -NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

3. Utilisation d'au moins une amine carbonylée de formule (I) ou de l'un de ses sels d'addition avec un acide, pour la préparation d'une composition de soin et/ou de traitement des fibres kératiniques d'être humain, destinée à induire et/ou stimuler la croissance des fibres kératiniques et/ou freiner leur chute et/ou augmenter leur densité, 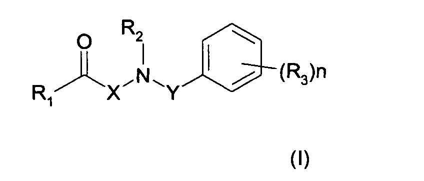 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
- un radical alkyle, linéaire ou ramifié, saturé ou insaturé, en C₁-C₆, ou un radical aryle,
- un atome d'halogène, ou
- un groupe choisi parmi -CN, -OR, -SR, -NRR', -COR, -COOR, -CONRR', -NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR,-COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', -NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

4. Utilisation selon l'une des revendications précédentes, **caractérisée en ce que** les fibres kératiniques sont des cheveux, des sourcils, des cils, des poils de barbe, de moustache et des poils pubiens.

5. Utilisation cosmétique d'au moins une amine carbonylée de formule (I) ou de l'un de ses sels d'addition avec un acide, dans une composition cosmétique de soin capillaire d'être humain, comme agent pour réduire la chute naturelle des cheveux et/ou augmenter leur densité et/ou traiter l'alopécie andro-chrono-génétique, 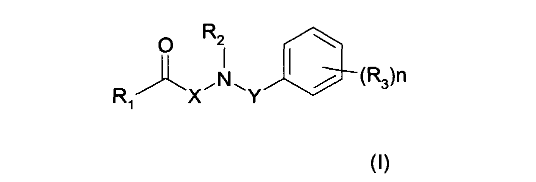 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
- un radical alkyle, linéaire ou ramifié, saturé ou insaturé, en C₁-C₆, ou un radical aryle,
- un atome d'halogène, ou
- un groupe choisi parmi -CN, -OR, -SR, -NRR', -COR, -COOR, -CONRR', -NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR,-COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', -NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

6. Utilisation d'au moins une amine carbonylée de formule (I) ou de l'un de ses sels d'addition avec un acide, pour la préparation d'une composition capillaire d'être humain, destinée à induire et/ou stimuler la croissance des cheveux et/ou freiner leur chute et/ou traiter l'alopécie androgénique, 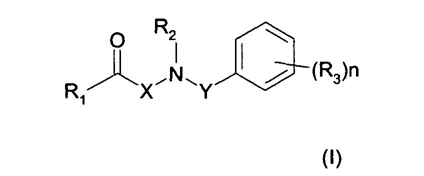 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
- un radical alkyle, linéaire ou ramifié, saturé ou insaturé, en C₁-C₆, ou un radical aryle,
- un atome d'halogène, ou
- un groupe choisi parmi -CN, -OR, -SR, -NRR', -COR, -COOR, -CONRR', -NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR, -COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', -NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

7. Utilisation cosmétique d'au moins une amine carbonylée de formule (I) ou de l'un de ses sels d'addition avec un acide, dans une composition cosmétique de soin et/ou de maquillage des cils d'être humain, pour induire et/ou stimuler la croissance des cils et/ou augmenter leur densité, 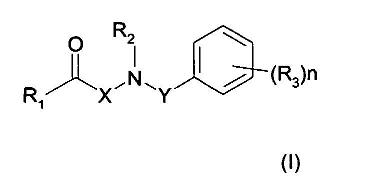 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
- un radical alkyle, linéaire ou ramifié, saturé ou insaturé, en C₁-C₆, ou un radical aryle,
- un atome d'halogène, ou
- un groupe choisi parmi -CN, -OR, -SR, -NRR', -COR, -COOR, -CONRR', -NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR,-COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', -NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

8. Utilisation d'au moins une amine carbonylée de formule (I) ou de l'un de ses sels d'addition avec un acide, pour la préparation d'une composition de soin et/ou de traitement des cils d'être humain, destinée à induire et/ou stimuler la croissance des cils et/ou augmenter leur densité, 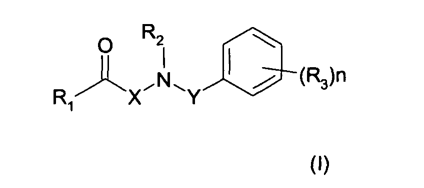 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
- un radical alkyle, linéaire ou ramifié, saturé ou insaturé, en C₁-C₆, ou un radical aryle,
- un atome d'halogène, ou
- un groupe choisi parmi -CN, -OR, -SR, -NRR', -COR, -COOR, -CONRR', -NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR, -COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', -NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

9. Utilisation d'au moins une amine carbonylée de formule (I) ou de l'un de ses sels d'addition avec un acide, pour la fabrication d'une composition destinée à traiter les désordres liés à une réduction de la microcirculation ou vascularisation cutanée et notamment d'un follicule pileux chez l'être humain, 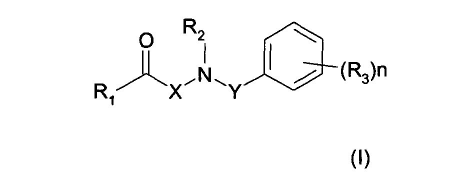 dans laquelle :
**a) R**_{**1**} est un radical alkyle, linéaire ou ramifié, saturé ou insaturé, en C₁-C₁₀ éventuellement substitué par un groupe oxo ou par au moins un groupement choisi parmi -OR, -SR, -NRR', -COR, -COOR, -CO-NRR', -NR-CO-R', F et CF₃ ;
**b) R**_{**2**} désigne un atome d'hydrogène ou un radical alkyle, linéaire ou ramifié, saturé ou insaturé, en C₁-C₈, éventuellement substitué par un groupe oxo ;
**c) R**_{**3**} désigne indépendamment :
- un radical alkyle, linéaire ou ramifié, saturé ou insaturé, en C₁-C₆, ou un radical aryle,
- un atome d'halogène, ou
- un groupe choisi parmi -CN, -OR, -SR, -NRR', -COR, -COOR, -CONRR', -NR-CO-R' et -CF₃ ;
**d) X** est une chaîne alkylène ou alkénylène en C₁-C₉, éventuellement substituée par un groupe oxo ou par au moins un groupement choisi parmi -OR, -SR, -NRR', -COR,-COOR, -CO-NRR', -NR-CO-R' et CF₃ et/ou un radical aryle ;
**e) Y** est une chaîne alkylène ou alkénylène en C₁-C₂₀, éventuellement substituée par au moins un groupement choisi parmi -OR, -SR, -NRR', -COOR, -CO-NRR', -NR-CO-R', F et CF₃ et/ou par un radical aryle ;
**f) n** est un entier allant de 0 à 5 ;
où R et R' désignent indépendamment un atome d'hydrogène ou un radical alkyle, linéaire ou ramifié, saturé ou insaturé en C₁-C₄.

10. Utilisation selon l'une des revendications précédentes, **caractérisée en ce que** l'amine carbonylée de formule (I) ou d'un de ses sels d'addition avec un acide est un composé répondant à l'une au moins des conditions suivantes :
• R₁ est un radical alkyle, linéaire ou ramifié, saturé ou insaturé, en C₁-C₅,
• R₂ est un atome d'hydrogène ou un radical alkyle saturé, en C₁-C₈, éventuellement substitué par un groupe oxo,
• R₃ est un radical alkyle, linéaire ou ramifié, saturé ou insaturé, en C₁-C₆, ou un groupe -OR où R est un atome d'hydrogène ou un radical alkyle linéaire ou ramifié, saturé, en C₁-C₄,
• n est égal à 0, 1 ou 2,
• X est une chaîne alkylène en C₁-C₄,
• Y est une chaîne alkylène en C₁-C₅.

11. Utilisation selon l'une des revendications précédentes, **caractérisée en ce que** l'amine carbonylée de formule (I) est telle que X est une chaîne éthylène.

12. Utilisation selon l'une des revendications précédentes, **caractérisée en ce que** l'amine carbonylée de formule (I) est telle que Y est une chaîne en C₁-C₃.

13. Utilisation selon l'une des revendications précédentes, **caractérisée en ce que** l'amine carbonylée répond à l'une des formules suivantes :

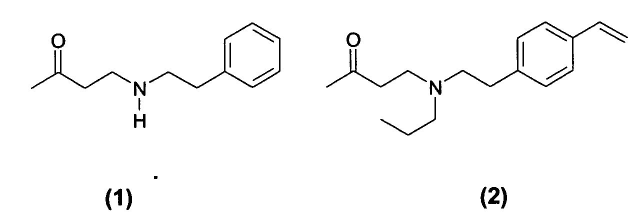

14. Utilisation selon l'une des revendications 1 à 11, **caractérisée en ce que** l'amine carbonylée répond à la formule (II) suivante :

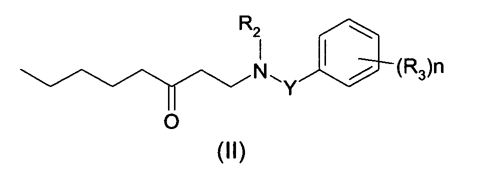

15. Utilisation selon la revendication 14, **caractérisée** en ce l'amine carbonylée de formule (II) ou son sel d'addition avec un acide satisfait à l'une au moins des conditions suivantes :
• R₂ désigne un atome d'hydrogène ou un radical alkyle, linéaire ou ramifié, saturé ou insaturé, en C₁-C₈, éventuellement substitué par un groupe oxo ;
• R₃ désigne
- un radical alkyle linéaire, ramifié ou cyclique, saturé ou insaturé, en C₁-C₆, ou un groupe aryle,
- un atome d'halogène, ou
- un groupe -CN, -OR, -SR, -NRR', -COR, -COOR, -CONRR' ou -CF₃ ;
• Y est une chaîne alkylène ou alkénylène en C₁-C₂₀, éventuellement substituée par au moins l'un des groupements -OR, -SR, -NRR', -COOR, -CO-NRR', -NR-CO-R', F et CF₃ et/ou par un groupe aryle,
• n est en entier allant de 0 à 5 ;
où R et R' désignent indépendamment un atome d'hydrogène ou un radical alkyle en C₁-C₄, linéaire ou ramifié.

16. Utilisation selon la revendication 15, **caractérisée en ce que** l'amine carbonylée satisfait à l'une au moins des conditions suivantes :
• R₂ est un atome d'hydrogène ou un radical alkyle, linéaire saturé, en C₁-C₈, éventuellement substitué par un groupe oxo,
• R₃ est un groupe -OR, où R est un atome d'hydrogène ou un radical alkyle, linéaire ou ramifié, saturé, en C₁-C₄,
• n est égal à 0, 1 ou 2,
• Y est une chaîne alkylène en C₁-C₅.

17. Utilisation selon la revendication 15 ou 16, **caractérisée en ce que** R₂ est un groupe alkyle en C₁-C₃.

18. Utilisation selon l'une des revendications 15 à 17, **caractérisée en ce que** n est égal à 0.

19. Utilisation selon l'une des revendications 15 à 18, **caractérisée en ce que** Y est une chaîne alkylène en C₁-C₃.

20. Utilisation selon l'une des revendications 15 à 19, **caractérisée en ce que** l'amine carbonylée répond à l'une des formules ci-dessous :

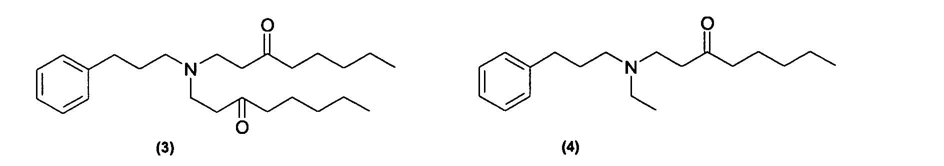

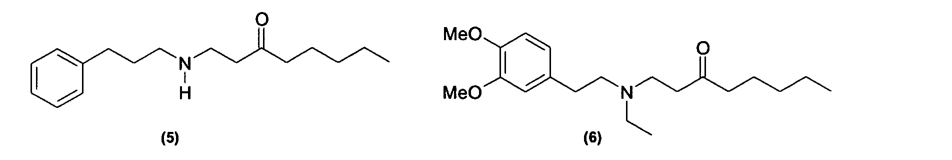

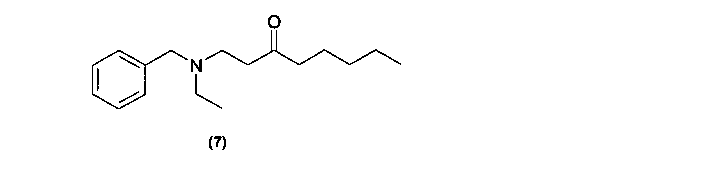

21. Utilisation selon l'une des revendications précédentes, **caractérisée en ce que** l'amine carbonylée de formule (I) ou un mélange d'amines de formule (I) est utilisé à une concentration allant de 10⁻³ à 10%, de préférence de 10⁻² à 5%, par rapport au poids total de la composition.

22. Utilisation selon l'une des revendications précédentes, **caractérisée en ce que** la composition est une composition à application topique.

23. Utilisation selon l'une des revendications précédentes, **caractérisée en ce que** la composition se présente sous forme de crème ou lotion capillaire, de shampooing, d'après-shampooing, de mascara capillaire ou pour cils.

24. Utilisation selon l'une des revendications précédentes, **caractérisée en ce que** la composition est sous forme de solution ou suspension aqueuse, alcoolique ou hydro-alcoolique.

25. Utilisation selon l'une des revendications précédentes, **caractérisée en ce que** la composition contient d'autres ingrédients choisis parmi les solvants, les épaississants ou gélifiants de phase aqueuse ou de phase huileuse, les matières colorantes solubles dans le milieu de la composition, les charges, les pigments, les antioxydants, les conservateurs, les parfums, les électrolytes, les neutralisants, les polymères filmogènes, les agents bloqueurs d'U.V., les actifs cosmétiques et pharmaceutiques autres que les composés de formule (I), leurs mélanges.

26. Utilisation selon l'une des revendications précédentes, **caractérisée en ce que** la composition contient au moins un composé additionnel actif favorisant la repousse et/ou limitant la chute des fibres kératiniques.

27. Utilisation selon la revendication 26, **caractérisée en ce que** le composé actif additionnel est choisi parmi l'aminexil, le 6-0-[(9Z,12Z)-octadéca-9,12-diènoyl]hexapyranose, les inhibiteurs de lipoxygénase, les inhibiteurs de bradykinine, les prostaglandines et leurs dérivés, les agonistes ou antagonistes des récepteurs des prostaglandines, les analogues non prostanoïques de prostaglandines, les vasodilatateurs, les antiandrogènes, les cyclosporines et leurs analogues, les antimicrobiens, les anti-inflammatoires, les rétinoïdes, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryle substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotérines, les cytokines et les promoteurs de facteurs de croissance, les inhibiteurs d'IL-1 ou d'IL-6, les promoteurs d'IL-10, les inhibiteurs de TNF, les vitamines, les benzophénones, l'hydantoïne, l'acide rétinoïque, les agents antiprurigineux, les antiparasitaires, les antifongiques, les agents antagonistes de calcium, les hormones, les triterpènes, les agents antiandrogènes, les inhibiteurs stéroïdiens ou non stéroïdiens des 5-α-réductases, les agonistes de canaux potassiques, les antagonistes du récepteur FP, leurs mélanges.

28. Utilisation selon l'une des revendications précédentes, **caractérisée en ce que** la composition contient, en outre, au moins un actif choisi parmi les protéines, les hydrolysats de protéine, les acides aminés, les polyols, l'urée, l'allantoïne, les sucres et dérivés de sucre, les vitamines hydrosolubles, les extraits végétaux, les hydroxy-acides, le rétinol, le tocophérol, les dérivés du rétinol ou du tocophérol, les acides gras essentiels, les céramides, les huiles essentielles, les dérivés de l'acide salicylique comme le n-octanoyl-5 salicylique, les esters des hydroxy-acides, les phospholipides, leurs mélanges.

29. Procédé cosmétique de traitement des fibres kératiniques humaines et/ou de la peau d'où émergent lesdites fibres, afin de stimuler la croissance des fibres kératiniques humaines, **caractérisé en ce qu'**il consiste à appliquer sur les fibres kératiniques humaines et/ou la peau, une composition cosmétique comprenant une quantité efficace d'au moins une amine carbonylée de formule (I) ou d'un de ses sels d'addition avec un acide, à laisser celle-ci en contact avec les fibres kératiniques et/ou la peau d'où émergent les fibres, et éventuellement à rincer les fibres kératiniques et/ou la peau.

30. Procédé cosmétique de traitement de l'alopécie andro-chrono-génétique, **caractérisé en ce qu'**il consiste à appliquer sur les cheveux humains et/ou le cuir chevelu, une composition cosmétique comprenant une quantité efficace d'au moins une amine carbonylée de formule (I) ou d'un de ses sels, à laisser celle-ci en contact avec les fibres kératiniques et/ou la peau, et éventuellement à rincer les fibres kératiniques et/ou la peau.

31. Procédé cosmétique de soin et/ou de maquillage des cils, **caractérisé en ce qu'**il consiste à appliquer sur les cils et/ou les paupières supérieures, une composition de mascara contenant une quantité efficace d'au moins une amine carbonylée de formule (I) ou d'un de ses sels d'addition avec un acide.
